# EUROPÄISCHE PATENTANMELDUNG

(11) **EP 1 964 513 A1**
(43) Veröffentlichungstag der Anmeldung: **03.09.2008**
(21) Anmeldenummer: 08102066.1
(22) Anmeldetag: 27.02.2008
(51) Int. Cl.: A61B 5/00

(54) **PCa-Nachweisgerät**

(30) Priorität: 01.03.2007 DE 102007010046
(71) Anmelder: SIEMENS AKTIENGESELLSCHAFT, 80333 München (DE)
(72) Erfinder: Fehre, Jens, 91353, Hausen (DE); Nanke, Ralf, 91077, Neunkirchen am Brand (DE)

(57) **Zusammenfassung**

Die Erfindung betrifft ein PCa-Nachweisgerät mit einem Messaufnehmer (11, 21) zur Detektion wenigstens eines PCa-spezifischen Biomarkers, einer Auswerteeinheit (12, 22) zur Ermittlung wenigstens eines Risiko-Wertes aus wenigstens einem vom Messaufnehmer (11, 21) detektierten PCa-spezifischen Biomarker und einer Anzeigeeinheit (13, 23) zur Anzeige wenigstens eines ermittelten Risiko-Wertes.

## Beschreibung

Die Erfindung betrifft ein PCa-Nachweisgerät.

Das Prostatakarzinom (PCa) weist eine steigende Indizenz auf und ist in den Industrieländern das häufigste Karzinom bei über 50 Jahre alten Männern. Bei frühzeitiger Erkennung existieren jedoch sehr gute Heilungschancen.

Für die Früherkennung von Prostatakarzinomen werden heutzutage DRU (Digital-rektale Untersuchung, Tastbefund) und PSA-Test (Prostataspezifisches Antigen, Bluttest) sowie TRUS (Transrektaler Ultraschall) eingesetzt.

Diese Methoden weisen sowohl einzeln als auch in Kombination nur eine unzureichende Sensitivität und Spezifität für Prostatakarzinome auf. Hinsichtlich PCa-Vorsorgeuntersuchungen gibt es derzeit keine von den Krankenkassen erstattete Vorsorgeuntersuchung, wie beispielsweise die Mammografieuntersuchung zur Krebsvorsorge bei Frauen. Lediglich bei einem konkreten Verdacht auf ein Prostatakarzinom werden die Kosten von der Krankenkasse übernommen, ansonsten besteht nur die Möglichkeit zur Selbstzahlung durch den Patienten, wie dies in größerem Umfang bereits bei PSA-Tests der Fall ist.

In der US 2004/0254445 A1 wird eine Untersuchung auf Vorliegen eines Prostatakarzinoms mittels Magnetresonanz-Bildgebung beschrieben. Bei dieser Untersuchung kann auch ein Kontrastmittel zum Einsatz kommen, das sich an der Prostata anlagert. Aufgrund der hohen Anschaffungs- und Betriebskosten eines Magnetresonanz-Gerätes kommt diese Untersuchung nicht für eine flächendeckende PCa-Untersuchung in Betracht.

Aus der US 2004/0053425 A1 und der US 2004/0072263 A1 ist jeweils ein tragbares PCa-Nachweisgerät bekannt, das eine Reaktionszelle aufweist, in der eine Gewebeprobe einer elektrochemischen Analyse unterzogen wird (In-vitro-Diagnose). Diese Gewebeprobe muss allerdings erst invasiv entnommen werden, z.B. durch eine Biopsie.

Aufgabe der vorliegenden Erfindung ist es, ein einfaches PCa-Nachweisgerät für einen sicheren Nachweis von Prostatakarzinomen zur Verfügung zu stellen.

Die Aufgabe wird erfindungsgemäß durch ein PCa-Nachweisgerät gemäß Anspruch 1 gelöst. Vorteilhafte Ausgestaltungen des erfindungsgemäßen PCa-Nachweisgerätes sind jeweils Gegenstand von weiteren Ansprüchen.

Das erfindungsgemäße PCa-Nachweisgerät umfasst
- einen Messaufnehmer zur Detektion wenigstens eines PCa-spezifischen Biomarkers,
- eine Auswerteeinheit zur Ermittlung wenigstens eines Risiko-Wertes aus wenigstens einem vom Messaufnehmer detektierten PCa-spezifischen Biomarker und
- eine Anzeigeeinheit zur Anzeige wenigstens eines ermittelten Risiko-Wertes.

Durch das erfindungsgemäße PCa-Nachweisgerät kann eine regelmäßige PCa-Vorsorgeuntersuchung bei Männern vorgenommen werden. Hierzu muss dem Patienten lediglich wenigstens ein PCa-spezifischer Biomarker verabreicht werden, der sich dann in den betreffenden Arealen der Prostata anreichert.

Die Verabreichung eines PCa-spezifischen Biomarkers kann durch eine direkte Injektion in die Prostata erfolgen. Eine systemische (intravenöse) oder eine orale Verabreichung ist ebenfalls möglich.

Der durch das PCa-Nachweisgerät nach Anspruch 1 detektierbare Biomarker (Kombination aus molekularem Marker, wie z.B. bestimmtes Protein oder Antikörper, und einer "Detektionskomponente", wie z.B. Bubble oder magnetischer Partikel) geht ausschließlich Bindungen mit bestimmten Molekülen ein, welche spezifisch für das Prostatakarzinom bzw. die zugehörige Angiogenese (Bildung neuer Blutgefäße aus bereits bestehenden Blutgefäßen) sind ("Molecular Tracking").

Die Detektierung wenigstens eines PCa-spezifischen Biomarkers mittels des erfindungsgemäßen PCa-Nachweisgerätes weist eine hohe Sensitivität und Spezifität auf. Sowohl der intrakorporale Messaufnehmer als auch der extrakorporale Messaufnehmer gewährleisten während der Untersuchung aufgrund der räumlichen Nähe zur Prostata eine zuverlässige Messung der Konzentration vorhandener PCa-spezifischer Biomarker und daraus resultierend eine zuverlässige Bestimmung eines PCa-Risikos.

Das erfindungsgemäße PCa-Nachweisgerät ist einfach anzuwenden und kann aufgrund der geringen Kosten flächendeckend bei niedergelassenen Ärzten eingesetzt werden.

Darüber hinaus treten bei Untersuchungen, die mit dem erfindungsgemäßen PCa-Nachweisgerät ausgeführt werden, keine Strahlenbelastungen, wie beispielsweise bei Röntgenuntersuchungen, auf.

Die mit dem PCa-Nachweisgerät nach Anspruch 1 durchführbare Untersuchungsmethode ist somit für eine systematische Früherkennung des PCa geeignet, womit ein zuverlässiges und kostengünstiges PCa-Screening bei einer großen Population möglich ist.

Übersteigt wenigstens einer der von der Auswerteeinheit ermittelten Risiko-Werte einen vorgegebenen Grenzwert, dann liegt ein positiver Befund vor und der Patient wird in eine Klinik zur urologischen Untersuchung überwiesen. In der Klinik können dann aufwändige Untersuchungen, z.B. Prostatastanzbiopsie oder die in der US 2004/0254445 A1 beschriebene Magnetresonanz-Untersuchung, durchgeführt werden.

Ein Biomarker, der durch das in Anspruch 1 beschriebene PCa-Nachweisgerät detektierbar ist, weist bestimmte physikalische und/oder chemische Eigenschaften auf, die seine Detektion in der Prostata ermöglicht. Beispielsweise kann ein für das erfindungsgemäße PCa-Nachweisgerät geeigneter Biomarker magnetische Partikel enthalten oder durch chemische Reaktionen Wärme erzeugen. Im Rahmen vorteilhafter Ausgestaltungen weist der Messaufnehmer des erfindungsgemäßen PCa-Nachweisgerätes hierzu beispielsweise einen Magnetsensor (Detektion magnetischer Signale) und/oder einen Temperatursensor (Erfassung thermischer Signale) und/oder einen optischen Sensor (Detektion luminiszierender Biomarker) und/oder einen akustischen Sensor (Erfassung akustischer Signale, ausgesandt von einem durch Ultraschall angeregten Biomarker, "Targeted Bubbles") auf.

Im Rahmen der Erfindung ist es möglich, den Messaufnehmer als intrakorporalen Messaufnehmer oder - bei ausreichender Stärke der Signale des PCa-spezifischen Biomarkers - als extrakorporalen Messaufnehmer auszuführen. Ein extrakorporaler Messaufnehmer ist hierbei auf der Körperoberfläche und möglichst nahe an der Prostata zu platzieren.

Da durch das erfindungsgemäße PCa-Nachweisgerät die Anreicherung der Prostata mit wenigstens einem PCa-spezifischen Biomarker detektiert wird, ist es besonders vorteilhaft, wenn der Messaufnehmer als intrakorporaler Messaufnehmer ausgebildet ist. In diesem Fall ist auch der Nachweis einer geringen Anreicherung der Prostata mit einem PCa-spezifischen Biomarker zuverlässig möglich.

Gemäß einer bevorzugten Ausführungsform des PCa-Nachweisgerätes ist der intrakorporale Messaufnehmer als Einmalartikel ausgebildet. Dies stellt eine wirkungsvolle Vorbeugung gegen Infektionsgefahren dar. Darüber hinaus wird der Sterilisationsaufwand eingespart, wodurch der Patientendurchsatz bei Reihenuntersuchungen deutlich erhöht werden kann.

Nachfolgend werden zwei Ausführungsbeispiele der Erfindung anhand der Zeichnung näher erläutert, ohne jedoch darauf beschränkt zu sein. Es zeigen:
- Fig. 1: eine erste Ausführungsform eines erfindungsgemäßen PCa-Nachweisgerätes in schematischer Schnittansicht,
- Fig. 2: eine zweite Ausführungsform des erfindungsgemäßen PCa-Nachweisgerätes in schematischer Schnittansicht.

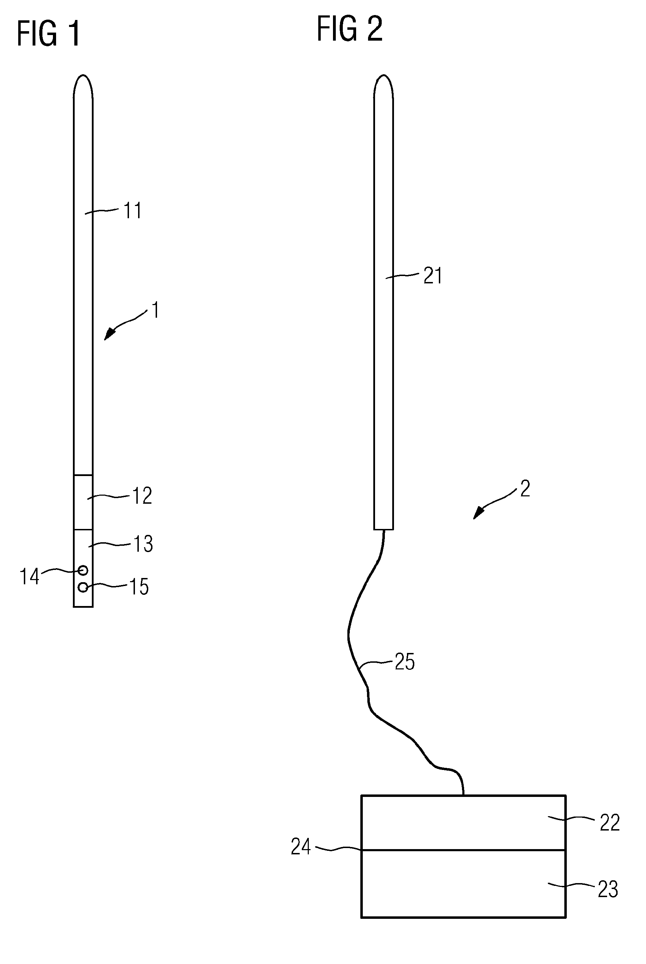

In Fig. 1 ist ein PCa-Nachweisgerät 1 mit einem Messaufnehmer 11 zur Detektion wenigstens eines PCa-spezifischen Biomarkers dargestellt. Der Messaufnehmer 11 ist im dargestellten Ausführungsbeispiel als intrakorporaler Messaufnehmer ausgeführt.

Das PCa-Nachweisgerät 1 umfasst weiterhin eine Auswerteeinheit 12 zur Ermittlung wenigstens eines Risiko-Wertes aus wenigstens einem vom intrakorporalen Messaufnehmer 11 detektierten PCa-spezifischen Biomarker sowie einer Anzeigeeinheit 13 zur Anzeige wenigstens eines ermittelten Risiko-Wertes.

Bei dem in Fig. 1 dargestellten Ausführungsbeispiel sind die Auswerteeinheit 12 und die Anzeigeeinheit 13 im intrakorporalen Messaufnehmer 11 integriert.

Aufgrund der daraus resultierenden geringen Baugröße für die Auswerteeinheit 12 und die Anzeigeeinheit 13 weist die Anzeigeeinheit 13 lediglich eine entsprechend kleine Anzeige auf. Hierfür besonders geeignet sind Leuchtdioden. Beispielsweise leuchtet eine grüne LED 14 bei einem negativen PCa-Befund (für den Patienten also positiv) und eine rote LED 15 bei Verdacht auf Prostatakarzinom, d.h. bei einem möglicherweise positiven Befund.

Der intrakorporale Messaufnehmer 11 kann als Einmalartikel ausgeführt sein. Dann ist es vorteilhaft, die Anzeigeeinheit 13 oder die Auswerteeinheit 12 und die Anzeigeeinheit 13 abnehmbar bzw. lösbar und damit wieder verwendbar auszuführen.

Im Rahmen der Erfindung ist es auch möglich, dass die Auswerteeinheit 12 funktional mit einer externen Anzeigeeinheit zusammenwirkt. Die Auswerteeinheit 12 ist dann nach der Untersuchung mit einer in Fig. 1 nicht dargestellten Anzeigeeinheit zu koppeln. Die Auswerteeinheit kann im Rahmen einer weiteren Ausgestaltung nur als Speichereinheit (in Fig. 1 nicht dargestellt) ausgeführt sein, in der die ermittelten Werte einer Biomarker-Konzentration lediglich gespeichert werden. Die gespeicherten Werte der Biomarker-Konzentration sind dann nach Beendigung der Untersuchung in einer Ausleseeinheit auslesbar und der Auswerteeinheit zuführbar.

In Fig. 2 ist ein weiteres PCa-Nachweisgerät, das mit 2 bezeichnet ist, dargestellt. Das PCa-Nachweisgerät 2 umfasst ebenfalls einen Messaufnehmer 21 zur Detektion wenigstens eines PCa-spezifischen Biomarkers. Der Messaufnehmer 21 ist im dargestellten Ausführungsbeispiel wiederum als intrakorporaler Messaufnehmer ausgeführt.

Das PCa-Nachweisgerät 2 umfasst weiterhin eine Auswerteeinheit 22 zur Ermittlung wenigstens eines Risiko-Wertes aus wenigstens einem vom intrakorporalen Messaufnehmer 21 detektierten PCa-spezifischen Biomarker sowie einer Anzeigeeinheit 23 zur Anzeige wenigstens eines ermittelten Risiko-Wertes.

Bei dem PCa-Nachweisgerät gemäß Fig. 2 sind - im Gegensatz zur integrierten Bauweise gemäß Fig. 1 - die Auswerteeinheit 22 und die Anzeigeeinheit 23 als separate Bauteile ausgeführt, die vorzugsweise in einem gemeinsamen Gehäuse 24 angeordnet sind. Aufgrund eines gegenüber einer Ausgestaltung nach Fig. 1 größeren Bauvolumens muss die Anzeige nicht notwendigerweise als LED-Anzeige ausgeführt sein. Vielmehr kann die Anzeige auch als analoge oder digitale Anzeige (LCD) ausgebildet sein. Auch ein Monitor ist im Rahmen der Erfindung als Anzeige möglich.

Bei der in Fig. 2 dargestellten Variante ist der intrakorporale Messaufnehmer 21 über ein Daten- und Versorgungskabel 25 mit der Auswerteeinheit 22 verbunden.

Der intrakorporale Messaufnehmer 21 kann auch bei dem PCa-Nachweisgerät 2 als Einmalartikel ausgeführt sein. Im Rahmen der Erfindung kann der intrakorporale Messaufnehmer 21 jedoch auch als wieder verwendbarer Messaufnehmer ausgestaltet sein. Zur Entsorgung bzw. zur Sterilisation ist der Messaufnehmer 21 hierzu vom Daten- und Versorgungskabel 25 lösbar.

Alternativ zu einer optischen Anzeige (LED 14 und 15, analoge oder digitale Anzeige) oder als Unterstützung einer optischen Anzeige kann ein akustisches Signal vorgesehen sein.

Wie aus der Beschreibung der Ausführungsbeispiele ersichtlich, ist das erfindungsgemäße PCa-Nachweisgerät einfach anzuwenden und kann aufgrund der geringen Kosten flächendeckend bei niedergelassenen Ärzten eingesetzt werden.

Die mit dem PCa-Nachweisgerät nach Anspruch 1 durchführbare Untersuchungsmethode ist somit für eine systematische Früherkennung des PCa geeignet, womit ein zuverlässiges und kostengünstiges PCa-Screening bei einer großen Population möglich ist.

## Patentansprüche

1. PCa-Nachweisgerät mit
- einem Messaufnehmer (11, 21) zur Detektion wenigstens eines PCa-spezifischen Biomarkers,
- einer Auswerteeinheit (12, 22) zur Ermittlung wenigstens eines Risiko-Wertes aus wenigstens einem vom Messaufnehmer (11, 21) detektierten PCa-spezifischen Biomarker und
- einer Anzeigeeinheit (13, 23) zur Anzeige wenigstens eines ermittelten Risiko-Wertes.

2. PCa-Nachweisgerät nach Anspruch 1, bei dem der Messaufnehmer (11, 21) als intrakorporaler Messaufnehmer ausgebildet ist.

3. PCa-Nachweisgerät nach Anspruch 1, bei dem der Messaufnehmer (11, 21) als extrakorporaler Messaufnehmer ausgebildet ist.

4. PCa-Nachweisgerät nach Anspruch 2, bei dem der intrakorporale Messaufnehmer (11, 21) endoskopartig ausgebildet ist.

5. PCa-Nachweisgerät nach Anspruch 2, bei dem der intrakorporale Messaufnehmer(11, 21) zylinderförmig ausgebildet ist.

6. PCa-Nachweisgerät nach Anspruch 2, bei dem der intrakorporale Messaufnehmer (11, 21) schlauchartig ausgebildet ist.

7. PCa-Nachweisgerät nach Anspruch 1, bei dem der Messaufnehmer (11, 21) einen Magnetsensor umfasst.

8. PCa-Nachweisgerät nach Anspruch 1 oder 7, bei dem der Messaufnehmer (11, 21) einen Temperatursensor umfasst.

9. PCa-Nachweisgerät nach Anspruch 1 oder 8, bei dem der Messaufnehmer (11, 21) einen optischen Sensor umfasst.

10. PCa-Nachweisgerät nach Anspruch 1 oder 9, bei dem der Messaufnehmer (11, 21) einen akustischen Sensor umfasst.

11. PCa-Nachweisgerät nach Anspruch 1, bei dem der Messaufnehmer (11, 21) als Einmalartikel ausgebildet ist.

12. PCa-Nachweisgerät nach Anspruch 1, bei dem der ermittelte Risiko-Wert analog anzeigbar ist.

13. PCa-Nachweisgerät nach Anspruch 1, bei dem der ermittelte Risiko-Wert digital anzeigbar ist.

14. PCa-Nachweisgerät nach Anspruch 1, 12 oder 13, bei dem der ermittelte Risiko-Wert in Form einer Grenzwert-Anzeige darstellbar ist.

15. PCa-Nachweisgerät nach Anspruch 1, bei dem die Auswerteeinheit (22) und die Anzeigeeinheit (23) als separate Einheiten ausgeführt sind.

16. PCa-Nachweisgerät nach Anspruch 1, bei dem die Auswerteeinheit (22) und die Anzeigeeinheit (23) als gemeinsame Einheit ausgeführt sind.

17. PCa-Nachweisgerät nach Anspruch 1, bei dem der Messaufnehmer (11, 21) lösbar angeordnet ist.
